# EUROPEAN PATENT APPLICATION

(11) **EP 4 803 130 A1**
(43) Date of publication of application: **09.09.2026**
(21) Application number: 26161472.1
(22) Date of filing: 27.02.2026
(51) Int. Cl.: A61M 39/12

(54) **MEDICAL TUBING SYSTEM HAVING FITTING WITH ANTI-TAMPER SLEEVE AND JACKET LOCK**

(30) Priority: 03.03.2025 US 202563765925 P
(71) Applicant: Flex-Trac, Inc., Exton, PA 19341 (US)
(72) Inventor: Breitinger, Brian, Exton, PA 19341 (US); Edler, Dave, Exton, PA 19341 (US); Knezich, Andrew, Exton, PA 19341 (US)
(74) Representative: Finch, Steven Caffall

(57) **Abstract**

A fitting for use with medical tubing including a corrugated, metal tube and an external jacket, the fitting including a body configured to receive the medical tubing; a sealing member for placement in a valley of the corrugated, metal tube, the sealing member including a sealing surface and an integrated jacket lock; an adaptor configured to engage the body, the adaptor including an adaptor sealing surface; and an anti-tamper sleeve configured to encompass the adaptor and the body; wherein upon assembly, the tubing is compressed between the adaptor sealing surface and the sealing surface and the jacket lock makes physical connection with the jacket.

## Description

### BACKGROUND

Medical piping within health care facilities was traditionally rigid, copper tubing meeting NPFA 99 health care facilities code. The health care facilities range from hospitals, ambulatory health care centers and clinics to medical and dental offices, nursing homes and limited care facilities. Rigid copper tubing can be difficult to install.

### SUMMARY

According to an embodiment, a fitting for use with medical tubing including a corrugated, metal tube and an external jacket, the fitting including a body configured to receive the medical tubing; a sealing member for placement in a valley of the corrugated, metal tube, the sealing member including a sealing surface and an integrated jacket lock; an adaptor configured to engage the body, the adaptor including an adaptor sealing surface; and an anti-tamper sleeve configured to encompass the adaptor and the body; wherein upon assembly, the tubing is compressed between the adaptor sealing surface and the sealing surface and the jacket lock makes physical connection with the jacket.

Optionally, the sealing surface may be positioned at a first end of the sealing member, the first end facing the adaptor, wherein the jacket lock is positioned at a second end of the sealing member, the second end opposite the first end.

Optionally, the jacket lock may include a plurality of fingers at the second end of the sealing member.

Optionally, each of the plurality of fingers may include at least one tooth configured to engage the jacket upon installation of the fitting on the medical tubing.

Optionally, a slot may be formed between each adjacent pair of fingers.

Optionally, a bend relief cut may be formed in an outside surface of the sealing member between the first end of the sealing member and the second end of the sealing member.

Optionally, the bend relief cut may comprise an annular groove formed in the outside surface of the sealing member.

Optionally, the body may include a first end facing the adaptor and a second end opposite the first end; the fitting further comprising an internal tapered surface at the second end of the body, the internal tapered surface positioned to contact the plurality of fingers upon installation of the fitting on the medical tubing.

Optionally, the body may have a smallest internal diameter at the second end.

Optionally, the adaptor may include external threads and the body may include internal threads, the adapter external threads configured to mate with the body internal threads.

Optionally, the anti-tamper sleeve may include an opening in a sidewall thereof; the body including a recess on an exterior surface thereof; the fitting further comprising a pin configured to be placed in the opening and the recess to secure the anti-tamper sleeve to the body.

Optionally, the recess may comprise a continuous groove formed in an exterior to the body.

Optionally, the adaptor may include a flange having a plurality of tapped holes; the body including a flange including a plurality of passages; the fitting further comprising a plurality of fasteners configured to pass through the plurality of passages and engage the plurality of tapped holes to secure the body to the adaptor.

Optionally, the anti-tamper sleeve may include an opening in a sidewall thereof; the body including a recess on an exterior surface thereof; the fitting further comprising a pin configured to be placed in the opening and the recess to secure the anti-tamper sleeve to the body.

Optionally, the recess may comprise a pocket formed in an exterior to the body.

Optionally, corrugated, metal tube compressed between the adaptor sealing surface and the sealing surface may comprise a double flare of tubing.

Optionally, the adaptor sealing surface may be curved.

According to another embodiment, a tubing system includes medical tubing including a corrugated, metal tube and an external jacket; and a fitting secured to the medical tubing, the fitting including: a body configured to receive the medical tubing; a sealing member for placement in a valley of the corrugated, metal tube, the sealing member including a sealing surface and an integrated jacket lock; an adaptor configured to engage the body, the adaptor including an adaptor sealing surface; and an anti-tamper sleeve configured to encompass the adaptor and the body; wherein the tubing is compressed between the adaptor sealing surface and the sealing surface and the jacket lock makes physical connection with the jacket.

Technical effects of embodiments of the disclosure include the ability to provide a flexible piping solution along with a tamper-proof construction.

The foregoing features and elements may be combined together in any desired combination, unless expressly indicated otherwise. These features and elements as well as the operation thereof will become more apparent in light of the following description and the accompanying drawings. It should be understood, however, that the following description and drawings are intended to be illustrative and explanatory in nature and non-limiting.

### BRIEF DESCRIPTION OF THE DRAWINGS

FIG. 1 is a cross-sectional view of a first fitting and medical tubing in an unassembled state, in a first example embodiment.
FIG. 2 is a cross-sectional view of a sealing member of the first fitting.
FIG. 3 is an end view of the sealing member.
FIG. 4 is a perspective view of the sealing member.
FIG. 5 is a cross-sectional view of the first fitting and medical tubing in an assembled state.
FIG. 6 is an end view of the first fitting and medical tubing in the assembled state.
FIG. 7 is a perspective view of the first fitting and medical tubing in the assembled state.
FIG. 8 is a cross-sectional view of a second fitting and medical tubing in an unassembled state, in a second example embodiment.
FIG. 9 is a cross-sectional view of the second fitting and medical tubing in an assembled state.
FIG. 10 is an end view of the second fitting and medical tubing in the assembled state.
FIG. 11 is a perspective view of the second fitting and medical tubing in the assembled state.

### DETAILED DESCRIPTION

Embodiments of the invention are related to the disclosure in U.S. patent publication number US20200398038, entitled CORRUGATED MEDICAL TUBING SYSTEM HAVING FITTING WITH ANTI-TAMPER SLEEVE, the entire contents of which are incorporated herein by reference.

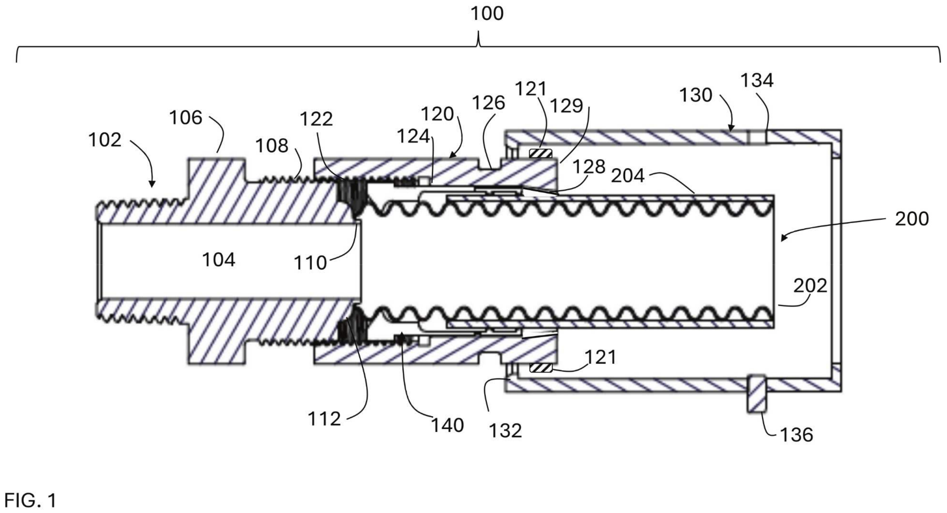

FIG. 1 is a cross-sectional view of a first fitting 100 and medical tubing 200 in an unassembled state, in an example embodiment. The medical tubing 200 includes an internal, corrugated, metal tube 202 and an external jacket 204. The corrugated, metal tube 202 may be made from phosphor bronze or another metal or an alloy. The corrugated, metal tube 202 has a series of peaks and valleys. The corrugated, metal tube 202 is corrugated in an annular manner. In example embodiments, the fitting 100 may be used with corrugated, metal tubes 202 having diameters of 1 inch or less.

The external jacket 204 has a smooth exterior. In an example embodiment, the jacket 204 is manufactured from low density polyethylene, but other materials that offer sufficient tensile strength to resist corrugation movement under pressure could be utilized. The jacket 204 may meet ASTM E84, with maximum Flame Spread index of 25 and maximum Smoke Density Index of 50. The jacket 204 may be coextruded with metal tube 202.

The first fitting 100 includes an adapter 102. The adaptor 102 may be made from brass or another metal or an alloy. The adaptor 102 includes an interior passage 104 along a longitudinal axis of the adaptor 102 for communicating a fluid (e.g., medical gas). Wrench flats 106 (e.g., hexagonal) are provided on the adaptor 102 to provide for tightening the adaptor 102 to a body 120. The adaptor 102 includes external threads 108 at first end of the adaptor 102. At a distal portion of the first end, the adaptor 102 includes a cylindrical, guiding diameter 110 that provides for centering an inner diameter of the medical tubing 200 on to adaptor 102. An adaptor sealing surface 112, adjacent to the guiding diameter 110, compresses a double flare of the corrugated, metal tube 202 between the adaptor 102 and a sealing member 140, as described in further detail herein. The adaptor sealing surface 112 may be curved (e.g., an arc) or a section of a sphere to provide a single line of contact with the corrugated, metal tube 202, thereby reducing the compression axial load required to create a seal.

The adaptor 102 is threaded into a body 120. The body 120 may be made from brass or another metal or an alloy. The body 120 include internal threads 122 configured to engage threads 108 on the adaptor 102. The body 120 includes an internal, annular shoulder 124 that extends inwards towards the sealing member 140, forming a reduced interior diameter of the body 120 at the shoulder 124. The shoulder 124 contacts the sealing member 140 when the fitting 100 is assembled, as described in further detail herein.

A recess 126 is formed in an exterior surface of the body 120. The recess 126 may be formed by an annular groove around the entire circumference of the outer surface of the body 120. Alternatively, the recess 126 may be formed by one or more pockets formed in the exterior surface of the body 120.

A second end of the body 120 includes an internal, tapered surface 128. The internal tapered surface 128 may be frustoconical such that an inner diameter of the body 120 decreases upon approaching a second distal end 129 of the body 120. In other words, at the second distal end 129 of the body 120, the inner diameter of the body 120 has its smallest value.

The first fitting includes an anti-tamper sleeve 130. The anti-tamper sleeve 130 is generally cylindrical, having two open ends, and a smooth exterior. The anti-tamper sleeve 130 may be made from stainless steel or another metal or an alloy. The body 120 includes one or more bumps 121 located on the exterior surface of the body 120 proximate to the second distal end 129 of the body 120. The bumps 121 are used to secure the anti-tamper sleeve 130 to the body 120 (e.g., for shipping of the fitting 100 and facilitating installation). The inner diameter 132 of the anti-tamper sleeve 130 is slightly less than the outer diameter of the bumps 121, but still allows the anti-tamper sleeve 130 to pass over the bumps 121. This retains the anti-tamper sleeve 130 to the body 120 and allows the anti-tamper sleeve 130 to rotate freely relative to the body 120 once the anti-tamper sleeve 130 is slid over the bumps 121. The inner diameter 132 of the anti-tamper sleeve 130 may be chamfered to facilitate sliding the anti-tamper sleeve 130 over the bumps 121. The bumps 121 on the body 120 may have a forward facing and/or rearward facing beveled surfaces to facilitate sliding the anti-tamper sleeve 130 over the bumps 121 and installing/removing the anti-tamper sleeve 130 from the body 120.

The anti-tamper sleeve 130 includes at least one opening 134 that passes through a wall of the anti-tamper sleeve 130. Once the anti-tamper sleeve 130 is positioned over the adaptor 102 and the body 120, a pin 136 may be installed in the opening(s) 134. The pin(s) 136 are received in the recess 126 to secure the anti-tamper sleeve 130 to the body 120.

The first fitting 100 includes a sealing member 140 having an integrated jacket lock. The sealing member 140 may be made from stainless steel or another metal or an alloy. The sealing member 140 is used to compress a corrugation of the corrugated, metal tube 202 between the sealing member 140 and the adaptor sealing surface 112. The sealing member 140 also provides a jacket lock, as described in further detail herein.

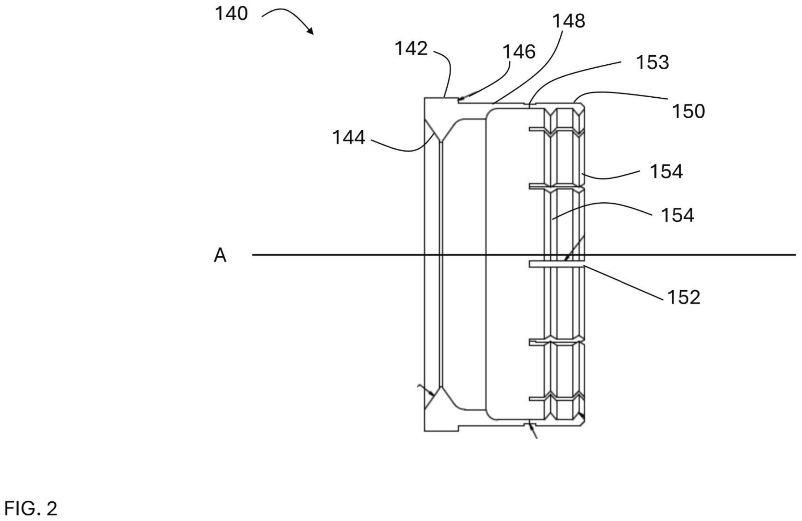

FIG. 2 is a cross-sectional view of the sealing member 140 having an integrated jacket lock, in an example embodiment. The sealing member 140 may be formed using a split assembly, with each segment having an interior as shown in FIG. 2. The sealing member 140 includes an annular ring 142 at a first end of the sealing member 140. A sealing surface 144 is provided at the inner diameter of the annular ring 142. The sealing surface 144 may be a frustoconical, thereby providing a flat surface opposite the adaptor sealing surface 112.

A backside of the annular ring 142 includes a mechanical stop 146. The mechanical stop 146 is formed by a reduction in the outer diameter from the annular ring 142 to a cylindrical core 148 of the sealing member 140. The mechanical stop 146 coacts with the body 120 when the fitting 100 is assembled, as described in further detail herein.

A second end of the sealing member 140 includes the integrated jacket lock having one or more fingers 150 that can flex or bend relative to the core 148. The one or more fingers 150 may be joined to the core 148 at a bend relief cut 153. The bend relief cut 153 is an annular groove formed in an outside surface of the core 148. The bend relief cut 153 facilitates flexing of the fingers 150 inwards towards a central axis, A, of the sealing member 140. A slot 152 is provided between adjacent fingers 150 to allow the fingers 152 to bend inwards towards the central axis, A, and to prevent the fingers 150 from interfering with each other.

The fingers 150 include one or more teeth 154, which may be formed by raised portions of material on the interior of the fingers 150. The teeth 154 may have a variety of shapes, including a triangular cross-section, pyramidical, barb-shaped, etc. When the first fitting 100 is assembled on the medical tubing 200, the teeth 154 are driven in to the jacket 204 to secure the fitting 100 to the medical tubing 200.

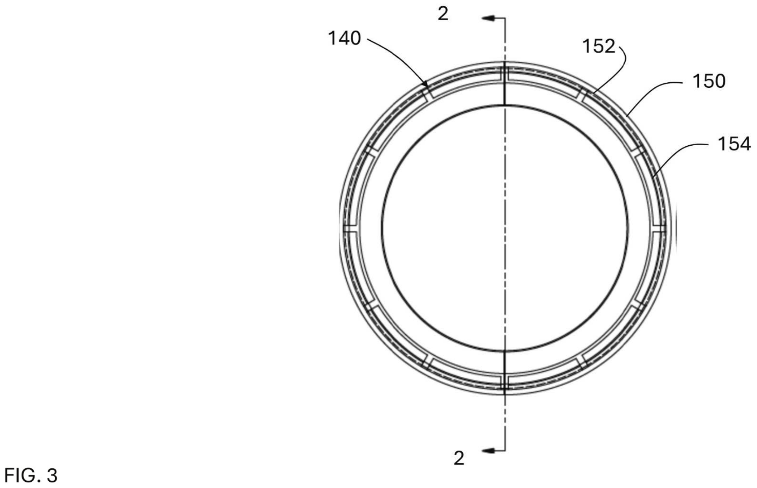

FIG. 3 is an end view of the sealing member 140 of FIG. 2. The sealing member 140 may be comprised of two sealing member segments, arranged in a split ring fashion. It is understood that the sealing member 140 may be implemented using different constructions, such as a collet or more than two segments.

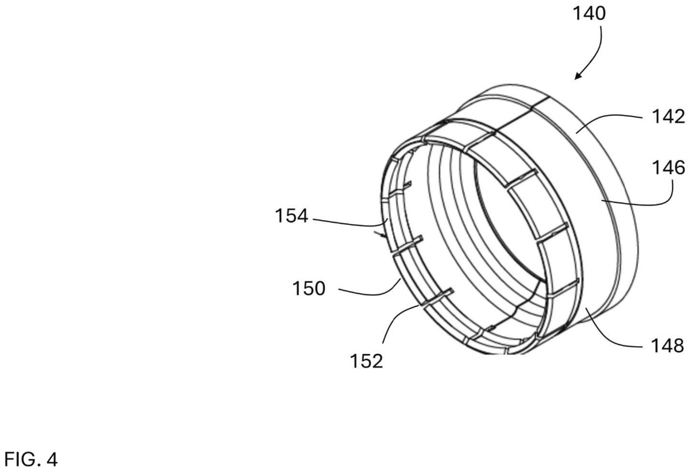

FIG. 4 is a perspective view of the sealing member 140 of FIG. 2.

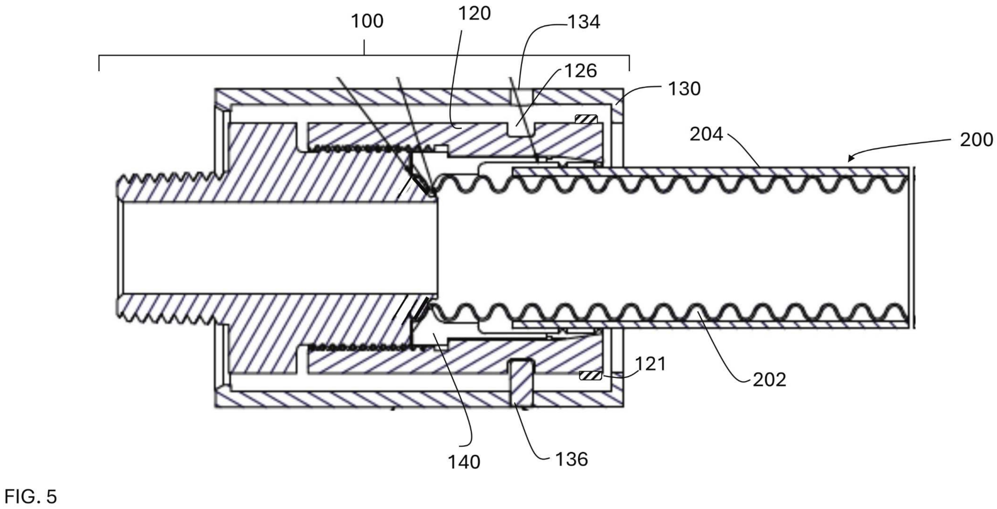

FIG. 5 is a cross-sectional view of the first fitting 100 and the medical tubing 200 in an assembled state, in an example embodiment. Assembly of the first fitting 100 and the medical tubing 200 may proceed as follows, although some steps may be performed in different order. A section of the jacket 204 is removed to expose at least one (e.g., three or four) convolution of the corrugated, metal tube 202. The medical tubing 200 is inserted through the anti-tamper sleeve 130 and the body 120. The sealing member 140 is placed about the corrugated, metal tube 202 so that the sealing surface 144 is positioned in the first valley of the corrugated, metal tube 202 with the sealing surface 144 facing the cut end of the corrugated, metal tube 202.

The adaptor 102 is threaded into the body 120 and tightened. As the adaptor 102 is threaded into the body 120, the peak of the corrugated, metal tube 202 between the sealing surface 144 and the adaptor sealing surface 112 is compressed to form a double flare of metal tubing between the adaptor and 102 and the body 120. During tightening, the shoulder 124 of the body 120 contacts the mechanical stop 146 of the sealing member 140 to drive the sealing member 140 towards the adapter 102.

As the adaptor 102 is threaded into the body 120, the internal tapered surface 128 of the body 120 travels over the fingers 150 of the sealing member 140. The reducing inner diameter of the internal tapered surface 128 drives the fingers 150 towards the jacket 204 of the medical tubing 200. The teeth 154 engage into and penetrate the jacket 204 to provide further mechanical connection between the medical tubing 200 and the fitting 100.

Once a suitable seal is formed between the adaptor 102 and the body 120, the anti-tamper sleeve 130 slid over the body 120 and the adaptor 102. The one or more pins 136 may be inserted through the one or more openings 134, each pin 136 engaging the recess 126. This provides a mechanical connection between the anti-tamper sleeve 130 and the body 120.

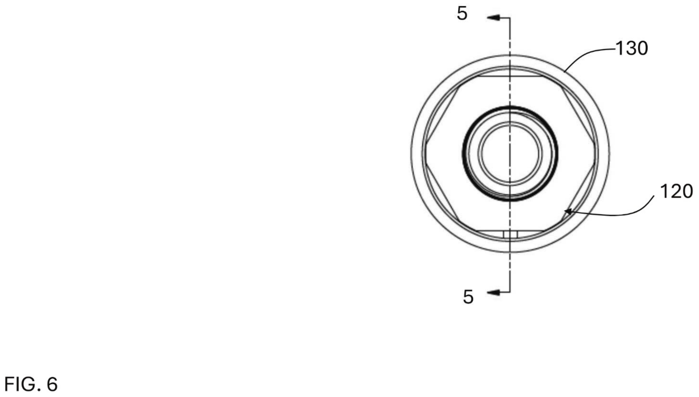

FIG. 6 is an end view of the first fitting 100 and the medical tubing 200 in an assembled state, in an example embodiment. As shown in FIG. 6, the anti-tamper sleeve 130 is positioned over the body 120.

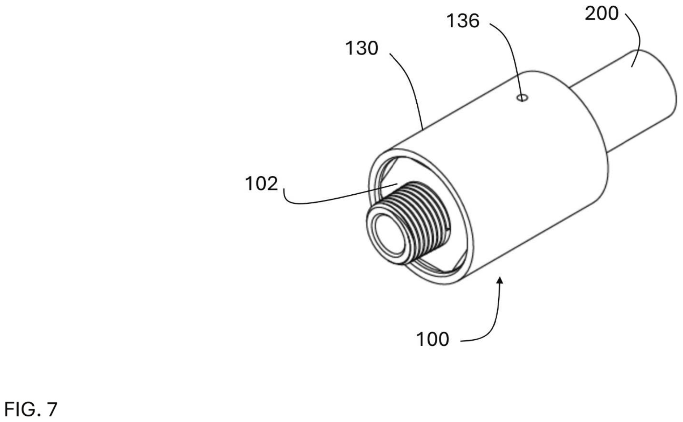

FIG. 7 is a perspective view of the first fitting 100 and the medical tubing 200 in an assembled state, in an example embodiment.

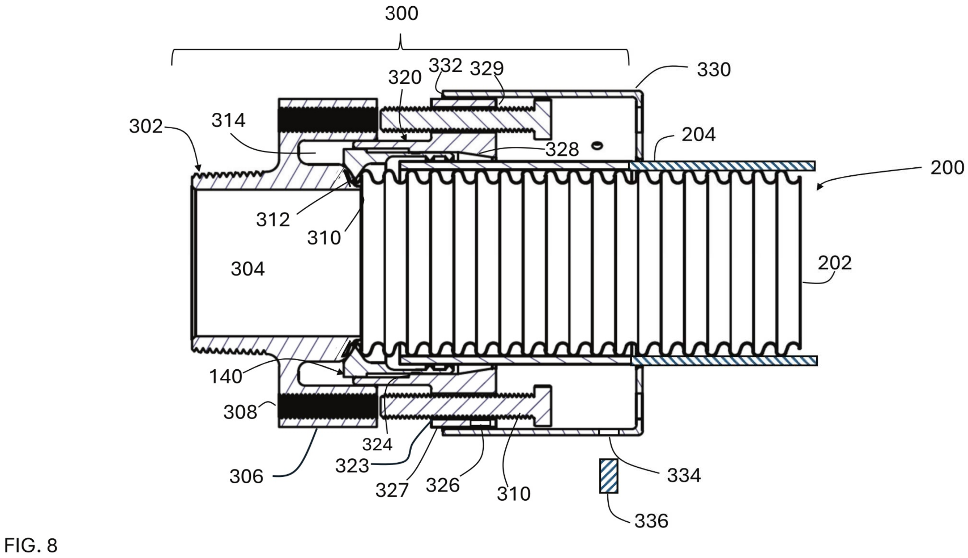

FIG. 8 is a cross-sectional view of a second fitting 300 and medical tubing 200 in an unassembled state, in a second example embodiment. In example embodiments, the second fitting 300 may be used with corrugated, metal tubes 202 having diameters greater than 1 inch.

The medical tubing 200 in FIG. 8 is similar to that in FIG. 1, and includes an internal, corrugated, metal tube 202 and an external jacket 204.

The second fitting 300 includes an adapter 302. The adaptor 302 includes an interior passage 304 along a longitudinal axis of the adaptor 302 for communicating a fluid (e.g., medical gas). A flange 306 extends radially outwardly from the adapter 302. The flange 306 includes a plurality of tapped holes 308 configured to receive fasteners 310 used to assemble the fitting 300.

At a distal portion of a first end, the adaptor 302 includes a cylindrical, guiding diameter 310 that provides for centering an inner diameter of the medical tubing 200 on to adaptor 302. An adaptor sealing surface 312 compresses a double flare of the corrugated, metal tube 202 between the adaptor 302 and a sealing member 140, as described in further detail herein. The adaptor sealing surface 312 may be curved (e.g., an arc) or a section of a sphere to provide a single line of contact with the corrugated, metal tube 202, thereby reducing the compression axial load required to create a seal.

The adaptor 302 receives a body 320 in an annular pocket 314 formed in the flange 306. A flange 327 extends radially outwardly from the body 320. The flange 327 includes a plurality of unthreaded passages 323 configured to receive fasteners 310 used to assemble the fitting 300.

The body 320 includes an internal, annular shoulder 324 that extends inwards towards the sealing member 140, forming a reduced interior diameter of the body 320 at the shoulder 324. The shoulder 324 contacts the sealing member 140 when the fitting 300 is assembled, as described in further detail herein.

A recess 326 is formed in an exterior surface of the body 320. The recess 326 may be formed by an annular groove formed around the entire circumference of the outer surface of the body 320. Alternatively, the recess 326 may be formed by individual pockets formed in the exterior surface of the body 320.

A second end of the body 320 includes an internal tapered surface 328. The internal tapered surface 328 may be frustoconical such that an inner diameter of the body 328 decreases upon approaching a second distal end 329 of the body 320. In other words, at the second distal end 329 of the body 320, the inner diameter of the body 320 has its smallest value.

An anti-tamper sleeve 330 is fitted over the body 320 and the adapter 302. The anti-tamper sleeve 330 is generally cylindrical and smooth on the exterior, having a first end with an opening sized to receive the body 320 and the adapter 302 and a second end having an opening sized to receive the medical tubing 200. The anti-tamper sleeve 330 may be made from a metal, such as stainless steel or an alloy. The anti-tamper sleeve 330 has an inner diameter 332 that may be slightly larger than an outer diameter of the adapter 302.

The anti-tamper sleeve 330 includes at least one opening 334 that passes through an outer wall of the anti-tamper sleeve 330. Once the anti-tamper sleeve 330 is positioned over the body 320, a pin 336 may be installed in the opening(s) 334. The pin(s) 336 are received in the recess 326 to secure the anti-tamper sleeve 330 to the body 320.

The second fitting 300 includes a sealing member 140 having an integrated, jacket lock. The sealing member 140 is used to compress a corrugation of the corrugated, metal tube 202 between the sealing member 140 and the adaptor sealing surface 312. The sealing member 140 also provides a jacket lock function. The sealing member 140 used in the second fitting 300 is similar in construction and functionality as the sealing member 140 used with the first fitting 100.

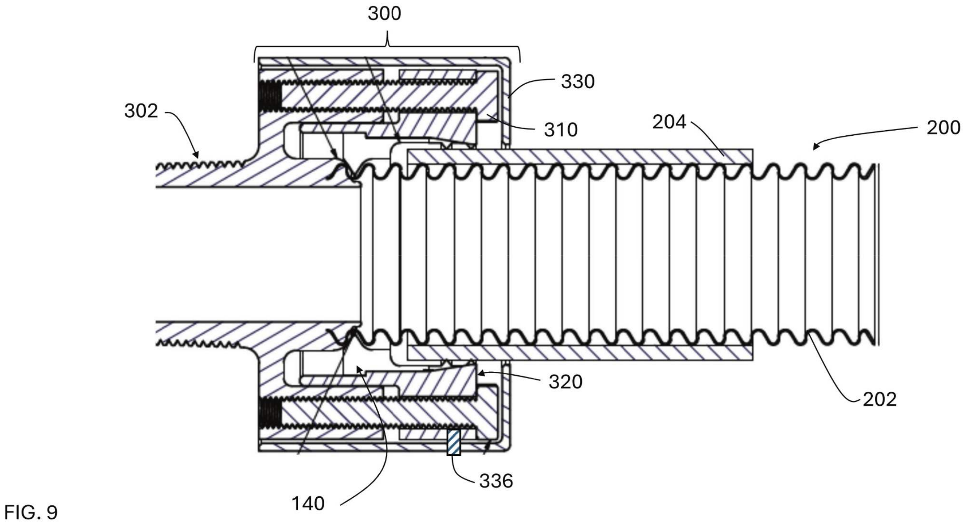

FIG. 9 is a cross-sectional view of the second fitting 300 and the medical tubing 200 in an assembled state, in an example embodiment. Assembly of the second fitting 300 and medical tubing 200 may proceed as follows, although some steps may be performed in different order. A section of the jacket 204 is removed to expose at least one convolution (e.g., three or four) of the corrugated, metal tube 202. The medical tubing 200 is inserted through the anti-tamper sleeve 330 and the body 320. The sealing member 140 is placed about the corrugated, metal tube 202 so that the sealing surface 144 is positioned in the first valley of the corrugated, metal tube 202 with the sealing surface 144 facing the cut end of the metal tube 202.

The adaptor 302 and the body 320 are drawn together by inserting the fasteners 310 through the passages 323 in the body 320 and into the tapped holes 308 in the adaptor 302. The fasteners 310 are then tightened. During tightening, the shoulder 324 of the body 320 contacts the mechanical stop 146 of the sealing member 140 to drive the sealing member 140 towards the adapter 302. As the body 320 and the adaptor 302 are drawn together, the peak of the metal tube 202 between the sealing surface 144 and the adaptor sealing surface 312 is compressed to form a double flare of metal tubing between the adaptor 302 and the body 320.

Also, as the body 320 and the adapter 302 are drawn together, the internal, tapered surface 328 of the body 320 travels over the fingers 150 of the sealing member 140. The reducing inner diameter of the internal, tapered surface 328 drives the fingers 150 towards the jacket 204 of the medical tubing 200. The teeth 154 engage into and penetrate the jacket 204 to provide mechanical connection between the medical tubing 200 and the fitting 300.

Once a suitable seal is formed between the adaptor 302 and the body 320, the anti-tamper sleeve 330 is slid over the body 320 and the adaptor 302. The one or more pins 336 may be inserted through the one or more openings 334, each pin 336 engaging the recess 326. This provides a mechanical connection between the anti-tamper sleeve 330 and the body 320.

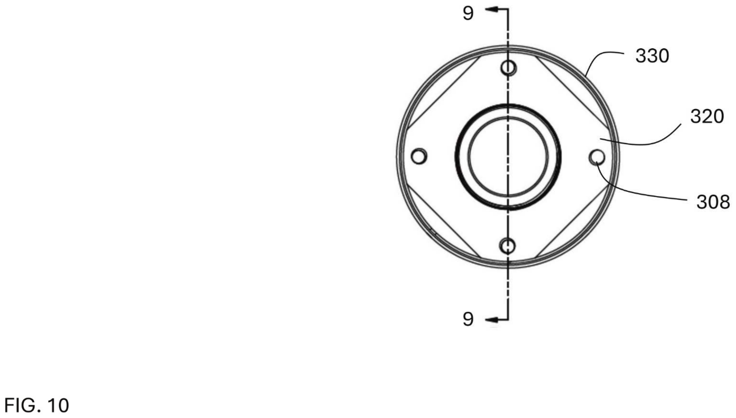

FIG. 10 is an end view of the second fitting 300 and medical tubing 200 in an assembled state, in an example embodiment.

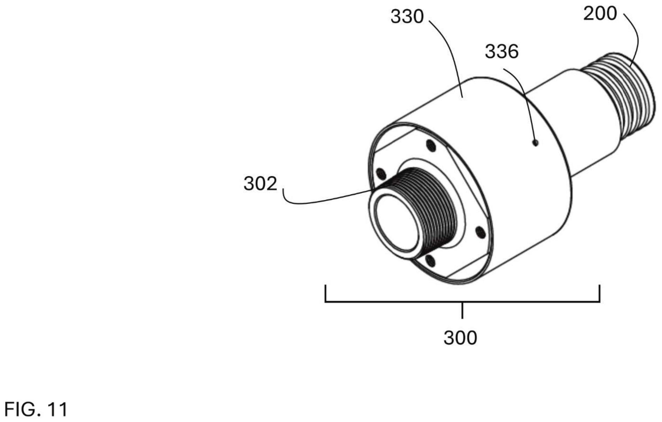

FIG. 11 is a perspective view of the second fitting 300 and a medical tubing 200 in an assembled state, in an example embodiment. Shown in FIG. 11 is a pin 336 positioned in the anti-tamper sleeve 330.

The medical tubing may be installed in a building, e.g. as a fixed installation, to transport medical fluids within the building. Medical fluids may include medical gases or liquids, which is to say, gases or liquids that are supplied to or drawn from the human body for medical purposes, e.g. during surgery or nursing care. By way of example, medical gases may include oxygen that is supplied to the body for respiration, or gases supplied to the body for anaesthesia. Where medical gases or liquids are to be drawn from the body, the medical tubing may be arranged to withstand external pressure so as to contain a vacuum, e.g. a partial vacuum, which can extract the gases or liquids to a collection or disposal location.

The terminology used herein is for the purpose of describing particular embodiments only and is not intended to be limiting of the present disclosure. As used herein, the singular forms "a", "an" and "the" are intended to include the plural forms as well, unless the context clearly indicates otherwise. It will be further understood that the terms "comprises" and/or "comprising," when used in this specification, specify the presence of stated features, integers, steps, operations, elements, and/or components, but do not preclude the presence or addition of one or more other features, integers, steps, operations, element components, and/or groups thereof.

Those of skill in the art will appreciate that various example embodiments are shown and described herein, each having certain features in the particular embodiments, but the present disclosure is not thus limited. Rather, the present disclosure can be modified to incorporate any number of variations, alterations, substitutions, combinations, sub-combinations, or equivalent arrangements not heretofore described, but which are commensurate with the scope of the present disclosure. Additionally, while various embodiments of the present disclosure have been described, it is to be understood that aspects of the present disclosure may include only some of the described embodiments. Accordingly, the present disclosure is not to be seen as limited by the foregoing description, but is only limited by the scope of the appended claims.

## Claims

1. A fitting for use with medical tubing including a corrugated, metal tube and an external jacket, the fitting comprising:
a body configured to receive the medical tubing;
a sealing member for placement in a valley of the corrugated, metal tube, the sealing member including a sealing surface and an integrated jacket lock;
an adaptor configured to engage the body, the adaptor including an adaptor sealing surface; and
an anti-tamper sleeve configured to encompass the adaptor and the body;
wherein upon assembly, the tubing is compressed between the adaptor sealing surface and the sealing surface and the jacket lock makes physical connection with the jacket.

2. The fitting of claim 1 wherein the sealing surface is positioned at a first end of the sealing member, the first end facing the adaptor,
wherein the jacket lock is positioned at a second end of the sealing member, the second end opposite the first end.

3. The fitting of claim 2 wherein the jack lock includes a plurality of fingers at the second end of the sealing member.

4. The fitting of claim 3 wherein the plurality of fingers each includes at least one tooth configured to engage the jacket upon installation of the fitting on the medical tubing.

5. The fitting of claim 3 further comprising a slot formed between each of plurality of fingers.

6. The fitting of claim 3 further comprising a bend relief cut formed in an outside surface of the sealing member between the first end of the sealing member and the second end of the sealing member.

7. The fitting of claim 6 wherein the bend relief cut comprises an annular groove formed in the outside surface of the sealing member.

8. The fitting of claim 3 wherein the body includes a first end facing the adaptor and a second end opposite the first end;
the fitting further comprising an internal tapered surface at the second end of the body, the internal tapered surface positioned to contact the plurality of fingers upon installation of the fitting on the medical tubing.

9. The fitting of claim 8 wherein the body has a smallest internal diameter at the second end.

10. The fitting of claim 1 wherein the adaptor includes external threads and the body includes internal threads, the adapter external threads configured to mate with the body internal threads.

11. The fitting of claim 10, wherein:
the anti-tamper sleeve includes an opening in a sidewall thereof;
the body includes a recess on an exterior surface thereof;
the fitting further comprising a pin configured to be placed in the opening and the recess to secure the anti-tamper sleeve to the body.

12. The fitting of claim 11 wherein the recess comprises a continuous groove formed in an exterior to the body.

13. The fitting of claim 1 wherein:
the adaptor includes a flange having a plurality of tapped holes;
the body includes a flange including a plurality of passages;
the fitting further comprising a plurality of fasteners configured to pass through the plurality of passages and engage the plurality of tapped holes to secure the body to the adaptor.

14. The fitting of claim 13, wherein:
the anti-tamper sleeve includes an opening in a sidewall thereof;
the body includes a recess on an exterior surface thereof;
the fitting further comprising a pin configured to be placed in the opening and the recess to secure the anti-tamper sleeve to the body.

15. The fitting of claim 14 wherein the recess comprises a pocket formed in an exterior to the body.

16. The fitting of claim 1 wherein corrugated, metal tube is compressed between the adaptor sealing surface and the sealing surface comprises a double flare of tubing.

17. The fitting of claim 1 wherein the adaptor sealing surface is curved.

18. A tubing system comprising:
medical tubing including a corrugated, metal tube and an external jacket; and
a fitting secured to the medical tubing, the fitting including:
a body configured to receive the medical tubing;
a sealing member for placement in a valley of the corrugated, metal tube, the sealing member including a sealing surface and an integrated jacket lock;
an adaptor configured to engage the body, the adaptor including an adaptor sealing surface; and
an anti-tamper sleeve configured to encompass the adaptor and the body;
wherein the tubing is compressed between the adaptor sealing surface and the sealing surface and the jacket lock makes physical connection with the jacket.
